# EUROPEAN PATENT APPLICATION

(11) **EP 2 402 050 A1**
(43) Date of publication of application: **04.01.2012**
(21) Application number: 10823270.3
(22) Date of filing: 15.09.2010
(51) Int. Cl.: A61M 25/00, A61B 1/00, A61M 25/01, A61B 1/005

(54) **FLEXIBLE MEDICAL TUBE AND INSERTION PART OF MEDICAL INSTRUMENT**

(30) Priority: 14.10.2009 JP 2009237634
(71) Applicant: OLYMPUS MEDICAL SYSTEMS CORP., Tokyo 151-0072 (JP)
(72) Inventor: AKUI, Nobuaki, Tokyo 192-8512 (JP)
(74) Representative: Schicker, Silvia
(86) International application number: PCT/JP2010/065901
(87) International publication number: WO 2011/046002

(57) **Abstract**

A medical flexible section (1) includes an inner tube body (6) which has a first bend allowing section (8) configured to allow bending in a predetermined direction provided on a side surface thereof and forms a duct and an outer tube body (7) which has a second bend allowing section (12) configured to allow bending in a predetermined direction provided on a side surface thereof and in which the inner tube body (6) is inserted. The medical flexible section (1) includes an operation ring (5) configured to change bendability of an insertion section (2) constituted of the outer tube body (7) and the inner tube body (6) by rotating the inner tube body (6) in a periaxial direction with respect to the outer tube body (7) to adjust a relative positional relationship between the first bend allowing section (8) and the second bend allowing section (12).

## Description

### Technical Field

The present invention relates to a medical flexible section and an insertion section of a medical apparatus that are inserted into a body cavity to be used.

### Background Art

In general, as medical instruments, there are a ureteral scope, a cannula for endoscopic retrograde cholangiopancreatography (ERCP), and others. An insertion section of each of such medical instruments is soft and flexible since forward and backward movements after insertion into a body cavity are taken into consideration. For example, in case of inserting the ureteral scope into a body cavity of a patient, the insertion section of the ureteral scope is guided to the renal pelvis from the bladder through the ureter. At this moment, since an end portion of the insertion section moves closer to the renal pelvis, the insertion section is pushed in while bending an end-side region of a bending portion of the ureteral scope.

Patent Document 1 discloses a bending section structure of a flexible tube of an endoscope. Here, V-shaped cut grooves are formed on a side surface of a multi-lumen tube. The cut grooves are alternately formed from directions substantially 180° different from each other. This bending section structure has characteristics that it can be readily bent in the directions along which the cut grooves are formed as compared with other directions.

Patent Document 2 discloses a catheter in which an inner tube made of a super-elastic alloy is combined with an outer tube made of silicone rubber. Here, a plurality of notches are arranged in the inner tube. As a result, the catheter is apt to bend in a predetermined direction.

Patent Document 3 discloses a medical bending tube. Here, a bending mechanism in which an inner sheath is combined with an outer sheath having notches is formed. An envelope is further put on the bending mechanism.
Patent Literature 1: Jpn. Pat. Appln. KOKAI Publication No. 2002-10971
Patent Literature 2: Jpn. Pat. Appln. KOKAI Publication No. 2002-180764
Patent Literature 3: Jpn. Pat. Appln. KOKAI Publication No. 2004-351005

### Disclosure of Invention

When an insertion section is soft and flexible, the insertion section can readily bend. Therefore, when the insertion section is pushed and inserted into an orifice (e.g., a ureteral orifice) of a patient, an operation of inserting an instrument including the insertion section straight from the orifice into a body may possibly become difficult due to insertion resistance during the inserting operation.

Thus, at the time of an operation of inserting the instrument into the orifice, a guide sheath or the like is also used to improve pushing performance of the insertion section. In this case, the guide sheath is inserted into the orifice in advance, and then an operation of inserting the insertion section into the guide sheath is carried out.

However, in this case, an operation of the medical instrument is complicated, and the number of necessary devices increases. Further, when a device like a stilet that is inserted into a tube to fix a shape is used, an inner space of the tube is occupied by the stilet. Therefore, there is a problem that an inner duct of the tube cannot be utilized during use of the stilet and a body fluid cannot be discharged.

Furthermore, an insertion section in which a coil compression type hardness variable structure like an existing GI endoscope is incorporated has been developed. However, in this case, a reduction in diameter of the insertion section is difficult, and it is difficult to mount this insertion section in a medical instrument such as a ureteral scope or a cannula.

Moreover, Patent Document 1 does not have a description about a structure that enables changing bending hardness of a multi-lumen tube (a bending tube) in particular.

Additionally, Patent Documents 2 and 3 do not have a description of a structure that enables changing the bending hardness either.

In view of the above-described problem, it is an object of the present invention to provide a medical flexible section and an insertion section of a medical apparatus that can change bendability as required when inserting into a body cavity and can simplify an operation of inserting into the body cavity without increasing the number of devices required for the insertion.

According to an aspect of embodiments, a medical flexible section comprising: an inner tube body which has a first bend allowing section configured to allow bending in a predetermined direction provided on a side surface thereof and forms a duct; an outer tube body which has a second bend allowing section configured to allow bending in a predetermined direction provided on a side surface thereof and in which the inner tube body is inserted; and a change section configured to change bendability of an insertion section constituted of the outer tube body and the inner tube body by rotating the inner tube body in a periaxial direction with respect to the outer tube body to adjust a relative positional relationship between the first bend allowing section and the second bend allowing section.

According to an aspect of embodiments, an insertion section of a medical apparatus, comprising: a bending section arranged at a distal end portion of the insertion section inserted into a body cavity; an operating section arranged at a proximal end portion of the insertion section; and a bending operating section configured to bend the bending section at the operating section, wherein the insertion section comprises: an outer tubular member; and an inner tubular member which is inserted in the outer tubular member to be rotatable in a periaxial direction, the inner tubular member has a first bend allowing section configured to allow bending in a predetermined direction at a distal end portion thereof, and the outer tubular member has a second bend allowing section configured to allow bending in a predetermined direction at a distal end portion thereof, the bending section is formed by assembling the first bend allowing section and the second bend allowing section, and the insertion section comprises a change section which is arranged in the operating section and changes bendability of the bending section by rotating the inner tubular member in the periaxial direction with respect to the outer tubular member to adjust a relative positional relationship of the first bend allowing section and the second bend allowing section.

### Brief Description of Drawings

FIG. 1 is a side view of an entire medical flexible section according to a first embodiment of the present invention;
FIG. 2A is a front view of an inner tube body of the medical flexible section according to the first embodiment;
FIG. 2B is a side view of the vicinity of an end portion of the inner tube body depicted in FIG. 2A;
FIG. 3A is a front view of an outer tube body of the medical flexible section according to the first embodiment;
FIG. 3B is a side view of the vicinity of an end portion of the outer tube body depicted in FIG. 3A;
FIG. 4 is a vertical cross-sectional view of the vicinity of the end portion of the medical flexible section according to the first embodiment;
FIG. 5 is a cross-sectional view taken through line 5-5 depicted in FIG. 4;
FIG. 6 is a side view showing a state that the medical flexible section according to the first embodiment is not bent;
FIG. 7 is a side view showing a state that the medical flexible section according to the first embodiment is bent;
FIG. 8 is a side view showing a state that an inner tube body of the medical flexible section according to the first embodiment is rotated 90°;
FIG. 9 is a cross-sectional view taken through line 9-9 depicted in FIG. 8;
FIG. 10A is a side view showing an outer tube body of a medical flexible section according to a second embodiment of the present invention;
FIG. 10B is a side view showing an inner tube body of the medical flexible section according to the second embodiment;
FIG. 11A is a cross-sectional view taken through line 11A-11A depicted in FIG. 10B;
FIG. 11B is a cross-sectional view taken through line 11B-11B depicted in FIG. 10B;
FIG. 12A is a perspective view showing an outer tube body of a medical flexible section according to a third embodiment of the present invention;
FIG. 12B is a perspective view of an inner tube body of the medical flexible section according to the third embodiment;
FIG. 13 is a side view of an endoscope according to a fourth embodiment of the present invention;
FIG. 14 is a side view showing the vicinity of a bending section of the endoscope according to the fourth embodiment;
FIG. 15 is a cross-sectional view taken through line 15-15 depicted in FIG. 14;
FIG. 16 is a side view showing the vicinity of a bending section of an endoscope according to a fifth embodiment of the present invention; and
FIG. 17 is a cross-sectional view taken through line 17-17 depicted in FIG. 16.

### Best Mode for Carrying Out the Invention

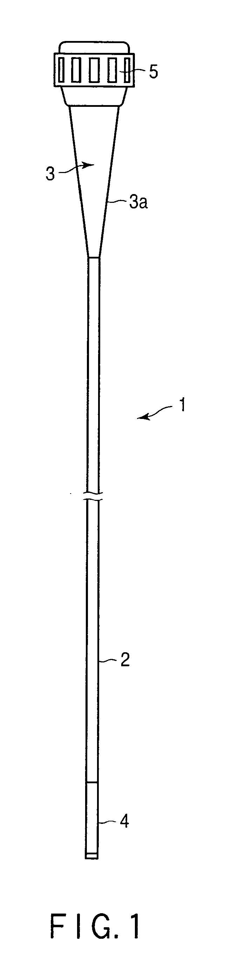

A first embodiment according to the present invention will now be described with reference to FIG. 1, FIG. 2A, FIG. 2B, FIG. 3A, FIG. 3B, FIG. 4, FIG. 5, FIG. 6, FIG. 7, FIG. 8, and FIG. 9. FIG. 1 shows a medical flexible section 1 according to this embodiment. The medical flexible section 1 is used for, e.g., an insertion section of a renal pelvis/ureteral scope or catheter. The medical flexible section 1 is, e.g., a medical apparatus. The medical flexible section 1 according to this embodiment has an elongated insertion section 2 inserted into a body and an operating section 3 which is coupled with a proximal end portion of this insertion section 2 and provided on an operator's hand side.

A diameter of the insertion section 2 is small. A bending change section 4 is provided at a distal end portion of the insertion section 2. The bending change section 4 (the distal end portion of the insertion section 2) can change the bendability between a state that bending is difficult (a hard state) and a state that bending is easy (a soft state) (the details will be described later). An operation ring (a change section) 5 is arranged on the operating section 3. The operation ring 5 is operated to change the bendability of the bending change section 4 (the distal end portion of the insertion section 2). The operation ring 5 is disposed to the operating section 3 so that the operation ring 5 can rotate in a periaxial direction of the insertion section 2.

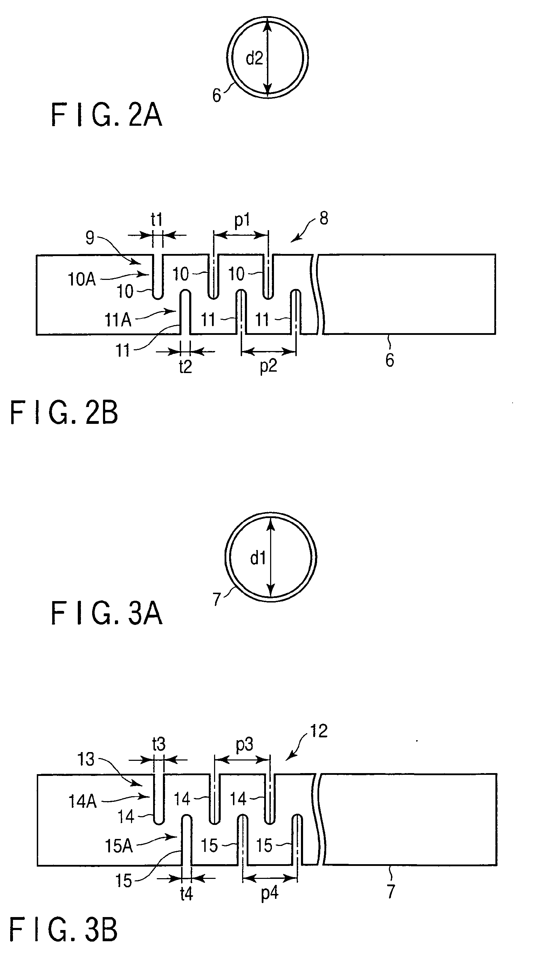

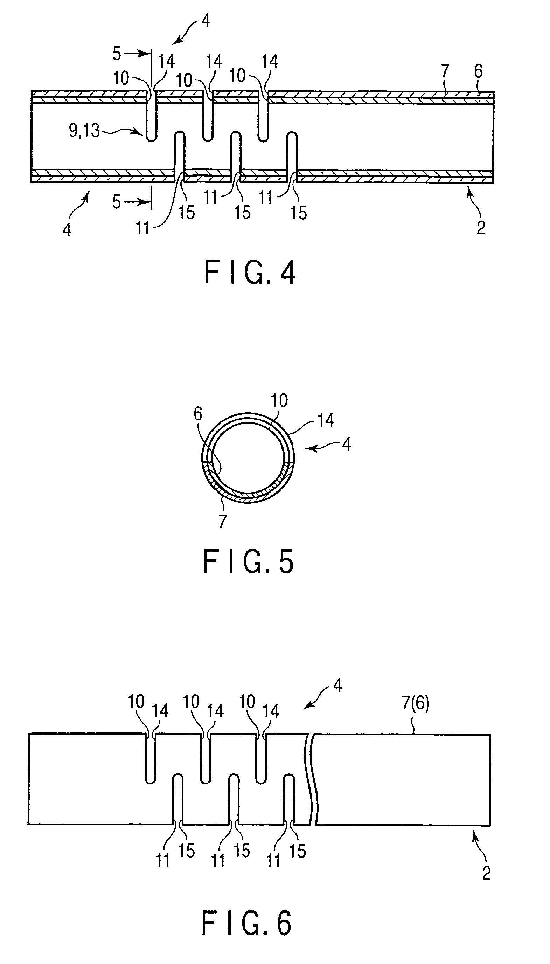

The insertion section 2 has an inner tube body 6 shown in FIG. 2A and FIG. 2B and an outer tube body 7 shown in FIG. 3A and FIG. 3B. The inner tube body 6 forms a duct of the medical flexible section 1. The inner tube body 6 is, e.g., a metal circular tube member. The outer tube body 7 is, e.g., a metal circular tube member. As shown in FIG. 4, the inner tube body 6 is inserted in the outer tube body 7. That is, the outer tube body 7 covers the inner tube body 6. An inside diameter d1 of the outer tube body 7 is slightly larger than an outside diameter d2 of the inner tube body 6. Therefore, as shown in FIG. 4, a clearance is formed between the outer tube body 7 and the inner tube body 6. The clearance has a size that enables the inner tube body 6 to rotate in the periaxial direction with respect to the outer tube body 7.

As shown in FIG. 2A and FIG. 2B, a first bend allowing section 8 that allows bending in a predetermined direction is provided on a side surface of the distal end portion of the inner tube body 6. In this embodiment, the first bend allowing section 8 has a first notch portion 9 formed in the side surface of the inner tube body 6. The first notch portion 9 has two slit string groups (a first slit string 10A and a second slit string 11A) formed in an outer peripheral surface at the distal end portion of the inner tube body 6.

As shown in FIG. 2A, FIG. 2B, and FIG. 5, the first slit string 10A is constituted by providing a plurality of substantially C-shaped slits 10 in parallel along the axial direction of the inner tube body 6. In detail, slits 10 are formed in along the axial direction of the inner tube body 6 in one side in the radial direction of the inner tube body 6 and in the side surface of the inner tube body 6. Further, each slit 10 is arranged (extended) in the direction orthogonal to the axial direction of the inner tube body 6. In slit 10, an appropriate groove width is t1, and each length (pitch) p1 between the adjacent slits 10 is aligned at a predetermined pitch. These slits 10 are opened in the same direction, respectively. These slits 10 form a slit group. Furthermore, each slit 10 is formed to be slightly larger than a substantially semicircular part (a part of 180°) of a circular cross section of the inner tube body 6 as shown in FIG. 5.

As shown in FIG. 2A and FIG. 2B, the second slit string 11A is constituted by providing a plurality of substantially C-shaped slits 11 in parallel along the axial direction of the inner tube body 6 like the first slit string 10A. In more detail, each slit 11 is formed in along the axial direction of the inner tube body 6 in the other side of the inner tube body 6 in the radial direction and in the side surface of the inner tube body 6. Moreover, each slit 10 is arranged (extended) in the direction orthogonal to the axial direction of the inner tube body 6. In slit 11, an appropriate groove width is t2, and each length (pitch) p2 between the adjacent slits 11 is aligned at a predetermined pitch. These slits 11 are opened in the same direction, respectively. These slits 11 form a slit group. Additionally, each slit 11 is formed to be slightly larger than a substantially semicircular part (a part of 180°) in a circular cross section of the inner tube body 6 like each slit 10.

It is to be noted that the number of slits 10 is equal to the number of slits 11 and t1=t2 and p1=p2 are achieved. Further, in the axial direction of the inner tube body 6, slits 10 and slits 11 are aligned (staggered) at an interval of a half pitch. That is, slits 10 and slits 11 are aligned in a staggered pattern along the axial direction of the inner tube body 6. Further, slits 10 and slits 11 are not arranged on the same straight line along the radial direction of the inner tube body 6.

Moreover, as shown in FIG. 3A and FIG. 3B, a second bend allowing section 12 that allows bending in a predetermined direction is formed on the side surface of the distal end of the outer tube body 7. In this embodiment, the second bend allowing section 12 has a second notch portion 13 formed in the side surface of the outer tube body 7. The second notch portion 13 has two slit strings (a first slit string 14A and a second slit string 15A) formed in an outer peripheral surface of the distal end portion of the outer tube body 7.

As shown in FIG. 3A, FIG. 3B, and FIG. 5, the first slit string 14A is constituted by providing a plurality of substantially C-shaped slits 14 in parallel along the axial direction of the outer tube body 7. In detail, slits 14 are formed in along the axial direction of the outer tube body 7 in one side of the radial direction of the outer tube body 7 and in the side surface of the outer tube body 7. Further, each slit 14 is arranged (extended) in the direction orthogonal to the axial direction of the outer tube body 7. In slit 14, an appropriate groove width is t3, and each length (pitch) p3 between the adjacent slits 10 is aligned at a predetermined pitch. These slits 14 are opened in the same direction, respectively. These slits 14 form a slit group.

As shown in FIG. 3A and FIG. 3B, the second slit string 15A is constituted by providing a plurality of substantially C-shaped slits 15 in parallel along the axial direction of the outer tube body 7 like the first slit string 14A. In more detail, each slit 15 is formed in along the axial direction of the outer tube body 7 in the other side in the radial direction of the outer tube body 7 and in the side surface of the outer tube body 7. Moreover, each slit 15 is arranged (extended) in the direction orthogonal to the axial direction of the outer tube body 7. In slit 15, an appropriate groove width is t4, and each length (pitch) p4 between the adjacent slits 15 is aligned at a predetermined pitch. These slits 15 are opened in the same direction, respectively. These slits 15 form a slit group.

It is to be noted that the number of slits 14 is equal to the number of slits 15 and t3=t4 and p3=p4 are achieved. Further, in the axial direction of the outer tube body 7, slits 14 and slits 15 are aligned (staggered) at an intervals of a half pitch. That is, slits 14 and slits 15 are aligned in a staggered pattern along the axial direction of the outer tube body 7. Further, slits 14 and slits 15 are not arranged on the same straight line along the radial direction of the outer tube body 7.

Furthermore, in this embodiment, as shown in FIG. 4, slits 10 and slits 11 are arranged at positions associated with slits 14 and slits 15 and formed with the same sizes with respect to slits 14 and slits 15.

The proximal end portion of the outer tube body 7 is fixed to the proximal end portion of a housing 3a of the operating section 3. Moreover, the proximal end portion of the inner tube section 6 is coupled with the non-illustrated coupling member disposed to the inside of the operation ring 5 of the housing 3a. As a result, when the operation ring 5 rotates in the periaxial direction of the insertion section 2, the inner tube body 6 is operated to rotate in the periaxial direction with respect to the outer tube body 7. When the operation ring 5 rotates, a rotational angle of the inner tube body 6 in the periaxial direction of the insertion section 2 varies in the range from, e.g., a fixed position of 0° to a rotational position of 90°.

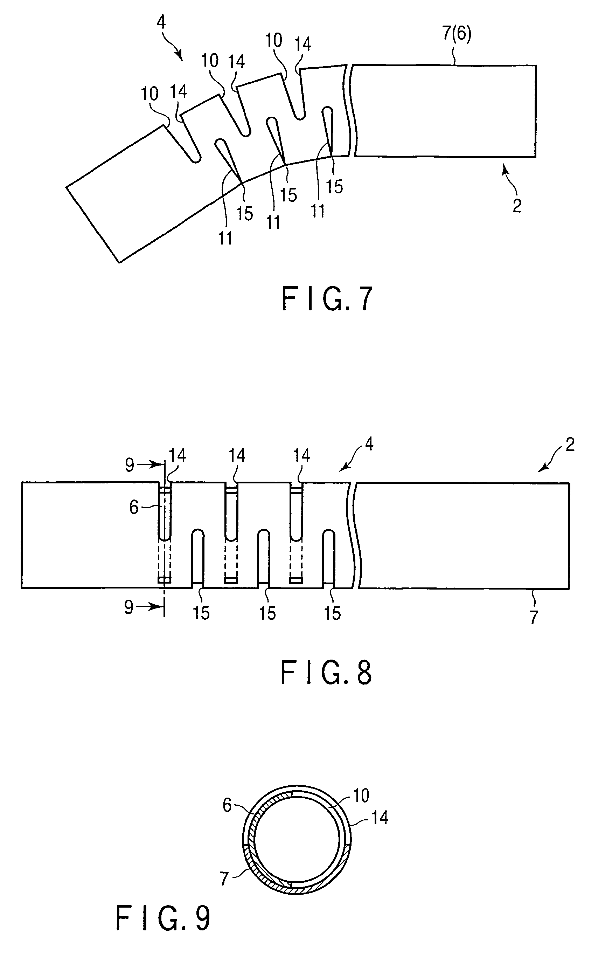

Moreover, when the operation ring 5 is held at the fixed position where the rotational angle is 0°, as shown in FIG. 4 and FIG. 5, the distal end portion (the bending change section 4) of the insertion section 2 is held at a first position where the first notch portion 9 (slits 10 and 11) of the inner tube body 6 overlaps the second notch portion 13 (slits 14 and 15) of the outer tube body 7. At this time, the distal end portion of the insertion section 2 changes to a state that it can readily bent in a predetermined direction deviated from the axial direction of the insertion section 2 (the soft state) as shown in FIG. 7 from a state that it is linearly stretched along the axial direction of the insertion section 2 as shown in FIG. 6. The predetermined direction means one of the direction of the second slit string 15A of the outer tube body 7 and the direction of the first slit string 14A of the same. That is, the predetermined direction means a bending direction along which the distal end of the insertion section 2 bends toward one of the first slit string 14A side and the second slit string 15A side.

Additionally, when the rotational angle is 90°, as shown in FIG. 8 and FIG. 9, the distal end portion (the bending change section 4) of the insertion section 2 is held (switched) at a second position where the first notch portion 9 (slits 10 and 11) of the inner tube body 6 does not overlap the second notch portion 13 (slits 14 and 15) of the outer tube body 7. At this time, as shown in FIG. 8, the distal end portion of the insertion section 2 is held in such a manner that a part of a tube wall of the inner tube body 6 overlaps the inside of each slit 15 of the outer tube body 7 and that a part of a tube wall of the inner tube body 6 overlaps the inside of each slit 14 of the outer tube body 7. In this case, the distal end portion of the insertion section 2 is in a state that it is hardly bent in a predetermined direction deviated from the axial direction of the insertion section 2 (a hard state) as shown in FIG. 8.

In this manner, the operation ring 5 operates the inner tube body 6 to rotate in the periaxial direction with respect to the outer tube body 7 and thereby adjusts a relative positional relationship between the first bend allowing section 8 and the second bend allowing section 12, whereby the bendability of the distal end portion (the bending change section 4) of the insertion section 2 constituted of the outer tube body 7 and the inner tube body 6 is changed.

Further, when the change section 5 operates the inner tube body 6 to rotate in the periaxial direction with respect to the outer tube body 7, the change section 5 operates the inner tube body 6 to move between the first position at which the first notch portion 9 and the second notch portion 13 are placed in substantially the same rotational direction and the second position at which the first notch portion 9 and the second notch portion 13 are placed in different rotational directions.

A function of this embodiment having the above configuration will now be described. When the medical flexible section 1 according to this embodiment is used, the operation ring of the operating section 3 rotates in the periaxial direction of the insertion section 2. As a result, the distal end portion (the bending change section 4) of the insertion section 2 changes to one of the unbendable state (the hard state) and the bendable state (the soft state). When the medical flexible section 1 according to this embodiment is used, the rotational angle is previously maintained at, e.g., the fixed position of 0°. In this case, the distal end portion of the insertion section 2 is held at the first position where the first notch portion 9 of the inner tube body 6 overlaps the second notch portion 13 of the outer tube body 7 (see FIG. 4 and FIG. 5). In this state, the bending change section 4 enters the state that the bending change section 4 can readily bend in a predetermined direction deviated from the axial direction of the insertion section 2 (the soft state) as shown in FIG. 7 from the state that the bending change section 4 is linearly stretched along the axial direction of the insertion section 2 as shown in FIG. 6.

In this state, the insertion section 2 is guided into the renal pelvis from the urinary bladder through the ureter. At this time, when the insertion section 2 is pushed and inserted into the urethral orifice, the bending change section 4 is apt to bend due to insertion resistance during the inserting operation. In this state, the operation of inserting the insertion section 2 straight into the orifice of a patient along the axial direction is hard to be performed.

Therefore, in this embodiment, before the insertion section 2 is inserted into the urethral orifice, the operation ring 5 is rotated to the rotational position of 90° in the periaxial direction of the insertion section 2. As a result, the bending change section 4 varies to the unbendable state (the hard state).

When the rotational angle is 90°, as shown in FIG. 8 and FIG. 9, the distal end portion (the bending change section 4) of the insertion section 2 is held (switched) at the second position where the first notch portion 9 of the inner tube body 6 does not overlap the second notch portion 13 of the outer tube section 7. At this time, the distal end portion of the insertion section 2 is held in a state that a part of the tube wall of the inner tube body 6 overlaps the inside of each slit 15 of the outer tube body 7 as shown in FIG. 8 and a state that a part of the tube wall of the inner tube body 6 overlaps the inside of each slit 14 of the outer tube body 7. In this state, the distal end portion of the insertion section 2 enters the state that the distal end portion of the insertion section 2 is hard to bend in a predetermined direction deviated from the axial direction of the insertion section 2 (the hard state) as shown in FIG. 8.

Then, the insertion section 2 is operated to be inserted into the ureteral orifice. At this time, when the insertion section 2 is pushed and inserted into the ureteral orifice, the bending change section 4 is hard to bend even though it undergoes the insertion resistance during the inserting operation. Therefore, in this state, the operation of inserting the insertion section 2 straight into the orifice of the patient is easily carried out. As a result, at the time of operation of guiding the insertion section 2 into the renal pelvis from the urinary bladder through the ureter, it is not necessary to additionally use a guide sheath and the like or use a device that is inserted into the tube to fix the shape like a stilet.

Therefore, in this embodiment, as compared with an example where the guide sheath or the like is additionally utilized to improve push-in performance of the insertion section 2 into the orifice of the patient and the insertion section 2 is thereby inserted, the number of devices required for insertion of the insertion section 2 is not increased, and the operation of inserting the insertion section 2 into the body cavity is simplified.

Thus, the above-described configuration exercises the following effects. That is, in the medical flexible section 1 according to this embodiment, when inserting the insertion section 2 into the body cavity, the bendability of the distal end portion (the bending change section 4) of the insertion section 2 can be changed as required. Therefore, as compared with the example where the guide sheath or the like is utilized to improve the push-in performance of the insertion section 2 and the insertion section 2 is thereby inserted, the operation of inserting the insertion section 2 into the body cavity can be simplified without increasing the number of devices required for the insertion of the insertion section 2 in this embodiment.

Additionally, the change section 5 can adjust a relative position of the inner tube body 6 (the first bend allowing section 8) and the outer tube body 7 (the first bend allowing section 9). As a result, in this embodiment, the distal end portion (the bending change section 4) of the insertion section 2 can be switched to one of the bendable state (the soft state, the rotational angle of 0°, the first position) and the unbendable state (the hard state, the rotational angle of 90°, the second position). As described above, in this embodiment, the bendability of the distal end portion (the bending change section 4) of the insertion section 2.

Further, in this embodiment, the distal end portion of the insertion section 2 can be bent in a predetermined direction by using the first bend allowing section 8 (the first notch portion 9, slits 10 and 11) and the first bend allowing section 12 (the second notch section 13, slits 14 and 15).

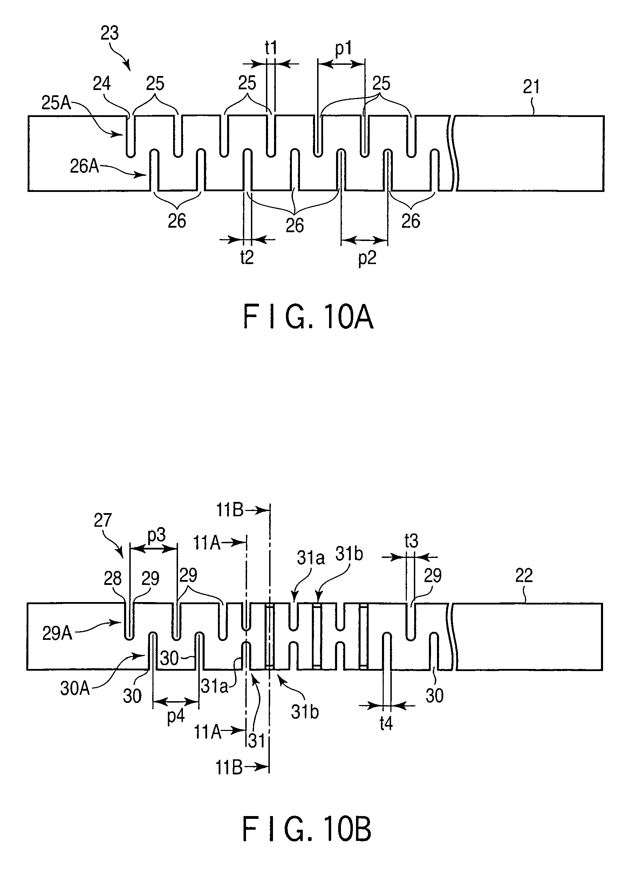

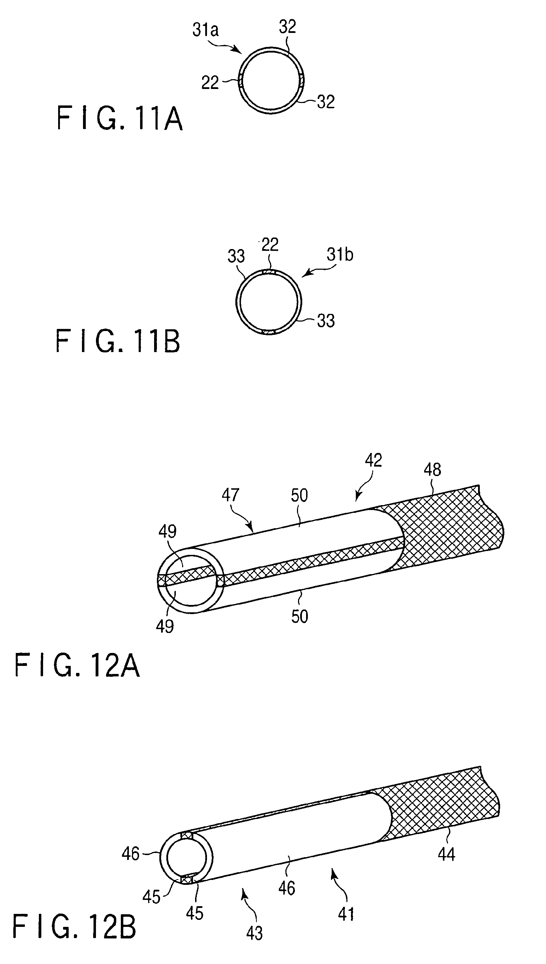

Furthermore, FIG. 10A, FIG. 10B, FIG. 11A, and FIG. 11B show a second embodiment of the present invention. This embodiment is obtained by changing the configuration of the bending change section 4 in the first embodiment (see FIGS. 1 to 9) as follows.

FIG. 10A shows an outer tube body 21 of an insertion section 2 in a medical flexible section 1 according to this embodiment, and FIG. 10B shows an inner tube body 22 of the insertion section 2 in the medical flexible section 1. As shown in FIG. 10A, a second bend allowing section 23 that allows bending in a predetermined direction is provided on the side surface of a distal end portion of the outer tube body 21. In this embodiment, the second bend allowing section 23 is constituted of a second notch portion 24 formed in the side surface of the outer tube body 21. The second notch section 24 has two slit strings (a first slit string 25A and a second slit string 26A) formed in an outer peripheral surface of the distal end portion of the outer tube body 21.

As shown in FIG. 10A, the first slit string 25A is constituted by providing a plurality of substantially C-shaped slits 25 along the axial direction of the outer tube body 21. In more detail, slits 25 are formed in along the axial direction of the outer tube body 21 in one side in the radial direction of the outer tube body 21 and in the side surface of the outer tube body 21. Further, each slit 25 is arranged (extended) in the direction orthogonal to the axial direction of the outer tube body 21. In slits 25, an appropriate groove width is t1, and each length (pitch) p1 between the adjacent slits 25 is aligned at a predetermined pitch. These slits 25 are opened in the same direction, respectively. These slits 25 form a slit group. Furthermore, each slit 25 is formed to be slightly larger than a substantially semicircular part (a part of 180°) in a circular cross section of the outer tube body 21.

As shown in FIG. 10A, the second slit string 26A is constituted by providing a plurality of substantially C-shaped slits 26 along an axial direction of the outer tube body 21 like slit string 25A. In more detail, slits 26 are formed in along the axial direction of the outer tube body 21 in the radial direction of the outer tube body 21 and in the side surface of the outer tube body 21. Moreover, each slit 26 is arranged (extended) in the direction orthogonal to the axial direction of the outer tube body 21. In slit 26, an appropriate groove width is t2, and each length (pitch) p2 between the adjacent slits 26 is aligned at a predetermined pitch. These slits 26 are opened in the same direction, respectively. These slits 26 form a slit group. Additionally, each slit 26 is formed to be slightly larger than a substantially semicircular part (a part of 180°) of the circular cross section of the outer tube body 21 like each slit 25 in the first slit string 25A.

It is to be noted that the number of slits 25 is equal to the number of slits 26 and t1=t2 and p1=p2 are achieved. Further, in the axial direction of the outer tube body 21, slits 25 and slits 26 are aligned (staggered) at an intervals of a half pitch.

As shown in FIG. 10B, a first bend allowing section 27 that allows bending in a predetermined direction is formed on a side surface of the distal end portion of the inner tube body 22. In this embodiment, the first bend allowing section 27 is constituted of a first notch portion 28 formed in the side surface of the inner tube body 22. The first notch portion 28 has two slit strings (a first slit string 29A and a second slit string 30A) formed in an outer peripheral surface at the distal end portion of the inner tube body 22.

As shown in FIG. 10B, the first slit string 29A is constituted by providing a plurality of substantially C-shaped slits 29 in parallel along the axial direction of the inner tube body 22. In detail, slits 29 are formed in along the axial direction of the inner tube body 22 in one side of the radial direction of the inner tube body 22 and in the side surface of the inner tube body 22. Further, each slit 29 is arranged (extended) in the direction orthogonal to the axial direction of the inner tube body 22. In slit 29, an appropriate groove width is t3, and each length (pitch) p3 between the adjacent slits 29 is aligned at a predetermined pitch. These slits 29 are opened in the same direction, respectively. These slits 29 form a slit group. Furthermore, each slit 29 is formed to be slightly larger than a substantially semicircular part (a part of 180°) of a circular cross section of the inner tube body 22.

As shown in FIG. 10B, the second slit string 30A is constituted by providing a plurality of substantially C-shaped slits 30 in parallel along the axial direction of the inner tube body 22 like the first slit string 29A. In more detail, each slit 30 is formed in along the axial direction of the inner tube body 22 in the other side in the radial direction of the inner tube body 22 and in the side surface of the inner tube body 22. Moreover, each slit 30 is arranged (extended) in the direction orthogonal to the axial direction of the inner tube body 22. In slit 30, an appropriate groove width is t4, and each length (pitch) p4 between the adjacent slits 30 is aligned at a predetermined pitch. These slits 30 are opened in the same direction, respectively. These slits 30 form a slit group. Additionally, each slit 30 is formed to be slightly larger than a substantially semicircular part (a part of 180°) in the circular cross section of the inner tube body 22 like each slit 29 in the first slit string 29A.

It is to be noted that the number of slits 29 is equal to the number of slits 30 and t3=t4 and p3=p4 are achieved. Further, in the axial direction of the inner tube body 22, slits 29 and slits 30 are aligned (staggered) at an intervals of a half pitch.

Further, as shown in FIG. 10B, in the inner tube body 22 according to this embodiment, a third slit string 31 having a configuration different from those of the first slit string 29A and the second slit string 30A is arranged at a central part of the first slit string 29A and the second slit string 30A in the axial direction of the inner tube body 22. The third slit string 31 has two types of slit portions (the first slit portion 31a and a second slit portion 31b) alternately arranged along the axial direction of the inner tube body 22.

As shown in FIG. 11A, the first slit portion 31a has two substantially C-shaped slits 32. Slits 32 are arranged in a circumferential wall surface of the inner tube body 22 and arranged (extended) in the direction (a vertical direction in FIG. 11A) orthogonal to the axial direction of the inner tube body 22. Each of the two slits 32 is formed to be slightly smaller than a substantially semicircular part (a part of 180°) of a circular cross section of the inner tube body 22.

As shown in FIG. 11B, the second slit portion 31b has two substantially C-shaped slits 33a. Slits 33 are arranged in the circumferential wall surface of the inner tube body 22 and arranged (extended) in the direction (a horizontal direction in FIG. 11B) orthogonal to the axial direction of the inner tube body 22. Each of the two slits 33 is formed to be slightly smaller than the substantially semicircular part (the part of 180°) of the circular cross section of the inner tube body 22.

Each first slit portion 31a is arranged at a position associated with slit 26 of the outer tube body 21. The second slit portion 31b is arranged at a position associated with slit 25 of the outer tube body 21.

A function of the configuration will now be described. When the rotational angle is held at the fixed position of 0°, the insertion section 2 of the medical flexible section 1 according to this embodiment is held at a first position where the first notch portion 28 of the inner tube body 22 overlaps the second notch portion 24 of the outer tube body 21. In this state, a bending change section 4 changes from a state that the bending change section 4 is linearly stretched along the axial direction of the insertion section 2 to a state that the bending change section 4 can readily bend in a predetermined direction deviated from the axial direction of the insertion section 2 (the soft state). The predetermined direction means one of a direction of the second slit string 26A of the outer tube body 21 and a direction of the first slit string 25A of the same.

Furthermore, when the operation ring 5 is rotated to a rotational position of 90° in the periaxial direction of the insertion section 2, the bending change section 4 changes to an unbendable state (the hard state). When this rotational angle is 90°, the distal end portion (the bending change section 4) of the insertion section 2 is held (switched) at a second position where the first notch portion 28 of the inner tube body 22 does not overlap the second notch portion 24 of the outer tube body 21. At this time, in the axial direction of the inner tube body 22, the same operation as that in the first embodiment is carried out at the parts except the central part of the first slit string 29A and the second slit string 30A. That is, the distal end portion of the insertion section 2 is held in a state that a part of a tube wall of the inner tube body 22 overlaps the inside of each slit 26 of the outer tube body 21 and a state that a part of the wall tube of the inner tube body 22 overlaps the inside of each slit 25 of the outer tube 21. In this case, the distal end portion of the insertion section 2 changes to a state that the distal end portion of the insertion section 2 is hardly bent in a predetermined direction deviated from the axial direction of the insertion section 2 (the hard state).

Furthermore, in this embodiment, at this time, the distal end portion of the insertion section 2 is held in a state each slit 32 of the inner tube body 22 overlaps the inside of each slit 26 of the outer tube body 21 and a state that each slit 33 of the inner tube body 22 overlaps the inside of each slit 25 of the outer tube body 21 at the central part of the first slit string 29A and the second slit string 30A of the inner tube body 22. In this case, the distal end portion of the insertion section 2 changes to a state that the distal end portion of the insertion section 2 can easily bend in a predetermined direction deviated from the axial direction of the insertion section 2 (the soft state).

Thus, the above-described configuration exercises the following effects. That is, in the medical flexible section 1 according to this embodiment, like the first embodiment, when inserting the insertion section 2 into a body cavity, the bendability of the distal end portion (the bending change portion 4) of the insertion section 2 can be changed as required.

In this embodiment, even if the insertion section 2 is changed to the unbendable state, a predetermined region (the central part of the first slit string 29A and the second slit string 30A [around slits 32 and 33]) can be bent, and the insertion section 2 can be pushed in according to a shape of an insertion target region, thereby simplifying the operation of inserting the insertion section 2 into the body cavity.

Furthermore, in this embodiment, when an operation ring 5 is rotated to the rotational position of 90° in the periaxial direction of the insertion section 2, the portion that the bending change section 4 is held in the unbendable state (the hard state) (both end sides of the bending change section 4) and the portion that the bending change section 4 is held in the bendable state (the soft state) (the central part of the bending change section 4 [around slits 32 and 33]) can be provided.

Moreover, FIG. 12A and FIG. 12B show a third embodiment. This embodiment is obtained by changing the configuration of the bending change state 4 in the first embodiment (see FIGS. 1 to 9) as follows.

As shown in FIG. 12B, a first bend allowing section 43 that allows bending in a predetermined direction is provided on a side surface of a distal end portion of an inner tube body 41.

In this embodiment, a tube main body 44 is formed in the inner tube body 41. The tube main body 44 is a resin tube made of a hard resin material. This resin material is, e.g., hard PVC, hard grade polyurethane, or nylon. Two first notch portions 45 are formed in a side surface of the distal end portion of the tube main body 44. The first notch portion 45 is obtained by notching both side surfaces of the distal end portion of the tube main body 44 into a substantially C-like shape in a circular cross section of the distal end portion. The first notch portions 45 are arranged along the axial direction of the inner tube body 41. Therefore, the distal end portion of the tube main body 44 is bifurcated.

A cover member (a first buried member) 46 is bonded to each first notch portion 45. The cover member 46 is made of a resin material softer than the tube main body 44. This resin material is a resin material which is of the same type as the tube main body 44 but softer than the tube main body 44. Such a resin material is, e.g., soft PVC, soft grade polyurethane, or nylon. As a result, adherence properties of the cover member 46 can be improved. The first bend allowing section 43 of the inner tube body 41 is formed by bonding the cover member 46 to each first notch portion 45. Further, as a method of forming the first bend allowing portion 43, when performing extrusion molding of the tube main body 44, a resin material forming the cover member 46 may be added to effect molding, thereby integrally molding the tube main body 44 and the cover member 46.

As shown in FIG. 12A, an outer tube body 42 has substantially the same configuration as the inner tube body 41. That is, a second bend allowing section 47 that allows bending in a predetermined direction is provided on a side surface of a distal end portion of the outer tube body 42.

In this embodiment, a tube main body 48 is formed in the outer tube body 42. The tube main body 48 is a resin tube made of a hard resin material. The resin material is, e.g., hard PVC, hard grade polyurethane, or nylon. Two second notch portions 49 are formed in the side surface of the distal end portion of the tube main body 48. The second notch portions 49 are obtained by notching both side surfaces of the tube main body 48 into a substantially C-like shape in a circular cross section of a cylinder of the tube main body 48. The second notch portions 49 are arranged along the axial direction of the outer tube body 42. Therefore, the distal end portion of the tube main body 48 is bifurcated.

A cover member (a second buried member) 50 is bonded to each second notch portion 49. The cover member 50 is formed of a resin material softer than the tube main body 48. This resin material is a resin material which is of the same type as the tube main body 44 and softer than the tube main body 44. Such a resin material is, e.g., soft PVC, soft grade polyurethane, or nylon. The second bend allowing section 47 of the outer tube body 42 is formed by bonding the cover member 50 to each second notch portion 49. As a method of forming the second bend allowing section 47, when performing extrusion molding of the tube main body 48, a resin material for forming the cover member 50 may be added to carry out molding, thereby integrally molding the tube main body 48 and the cover member 50.

Further, at the time of driving the operation ring 5 to rotate, the inner tube body 41 is operated to swivel in the range from a fixed position that a rotational angle of the insertion section 2 in the periaxial direction is 0° to a rotational position of 90°, for example.

Further, in the medical flexible section 1 according to this embodiment, when the rotational angle is held at the fixed position of 0°, the distal end portion of the insertion section 2 is held at a first position where the cover member 46 of the inner tube body 41 overlaps the cover member 50 of the outer tube body 42. At this time, the distal end portion of the insertion section 2 is changed to a state that it is apt to bend in a predetermined direction deviated from the axial direction of the insertion section 2 (a soft state) from a state that it is linearly stretched along the axial direction of the insertion section 2.

Furthermore, when the rotational angle is 90°, at a second position where the cover member 46 of the inner tube body 41 does not overlap the cover member 50 of the outer tube body 42, the distal end portion of the insertion section 2 is held (switched). At this time, the distal end portion of the insertion section 2 is held in a state that a part of a tube wall of the inner tube body 41 overlaps the inside of the cover member 50 of the outer tube body 42. In this case, the distal end portion of the insertion section 2 is hard to bend in a predetermined direction deviated from the axial direction of the insertion section 2.

As described above, when the operation ring 5 operates the inner tube body 41 to rotate in the periaxial direction with respect to the outer tube body 42, the operation ring 5 moves the inner tube body 41 between the first position that the cover member 46 of the inner tube body 41 is placed in substantially the same rotational direction as that of the cover member 50 and the second position that the cover member 46 is placed in the rotational direction different from that of the cover member 50.

Thus, the above-described configuration exercises the following effect. That is, in the medical flexible tube 1 according to this embodiment, when inserting the insertion section 2 into a body cavity, the bendability of the distal end portion (the bending change section 4) of the insertion section 2 can be changed as required. Therefore, as compared with an example that a guide sheath or the like is additionally utilized to improve push-in performance of the insertion section 2 and the insertion section 2 is thereby inserted, the operation of inserting the insertion section 2 into a body cavity can be simplified without increasing the number of devices required for insertion of the insertion section 2 in this embodiment.

Moreover, in this embodiment, when the tube main body 44 and the cover member 46 are made of the above-described resin material, adherence properties of the tube main body 44 and the cover member 46 can be improved.

Additionally, in this embodiment, when the tube main body 48 and the cover member 50 are made of the above-described resin material, adherence properties of the tube main body 48 and the cover member 50 can be improved.

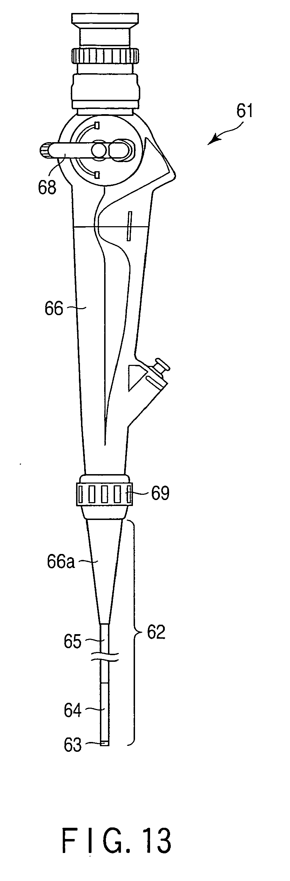

Further, FIG. 13, FIG. 14, and FIG. 15 show a fourth embodiment according to the present invention. This embodiment is applied to an insertion section 62 of an endoscope 61 in place of the medical flexible section 1 according to the first embodiment (see FIGS. 1 to 9). Such an endoscope 61 is a medical apparatus, and it is, e.g., a ureteral scope.

That is, the endoscope 61 has an elongated insertion section 62 that is inserted into a body cavity as shown in FIG. 13. This insertion section 62 has a distal end hard section 63, a bending section 64 that is operated to bend in two directions, and a flexible tube section 65 which is long and has flexibility. The insertion section 62 is formed by sequentially coupling the distal end hard section 63, the bending section 64, and the flexible tube section 65 from the distal end side of the insertion section 62. A non-illustrated illumination window portion through which illumination light is emitted, a non-illustrated observation window portion, and others are arranged in the distal end hard section 63.

A proximal end portion of the insertion section 62 is connected with a distal end portion of an operating section 66. A bending lever 68 configured to operate and bend the bending section 64 is arranged on a proximal end side of the operating section 66. Additionally, an operation ring (a change section) 69 that changes the bendability of the bending section 64 is arranged on the operating section 66. The operation ring 69 is disposed to the operating section 66 to be rotatable in the periaxial direction of the insertion section 62.

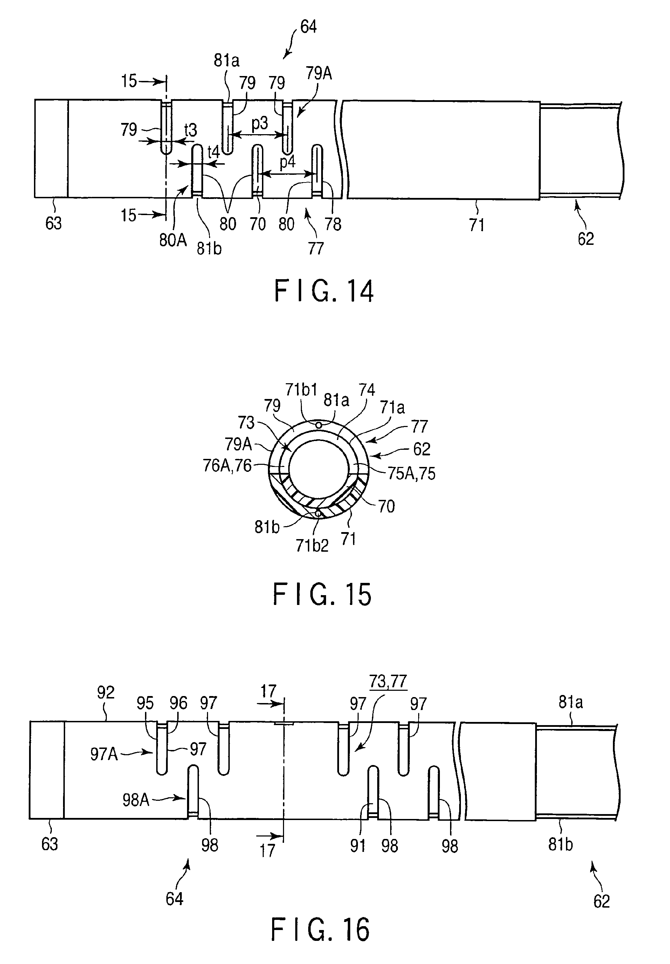

The insertion section 62 has an inner tubular member 70 and an outer tubular member 71 as shown in FIG. 14 and FIG. 15. The bending section 64 is formed by assembling the inner tubular member 70 and the outer tubular member 71. The inner tubular member 70 is, e.g., a resin tube. The outer tubular member 71 is, e.g., a resin multi-lumen tube. The outer tubular member 71 has a circular hole 71a through which the inner tubular member 70 is inserted and two small circular holes 71b1 and 71b2 formed in a circumferential wall portion of this circular hole 71a. Further, the inner tubular member 70 is inserted in the circular hole 71a of the outer tubular member 71. The two small circular holes 71b1 and 71b2 are arranged at positions which are 180° apart from each other in the circumferential direction of the outer tubular member 71.

An inside diameter of the circular hole 71a is formed to be slightly larger than an outside diameter of the inner tubular member 70. As a result, a clearance is formed between the circular hole 71a and the inner tubular member 70. This clearance has a size that enables the inner tubular member 70 to rotate in the periaxial direction with respect to the circular hole 71a.

The inner tubular member 70 has the same configurations as the inner tube body 6 and the like.

Here, a first bend allowing section 73 that allows bending in a predetermined direction is provided on a side surface of a distal end portion of the inner tubular member 70. The first bend allowing section 73 according to this embodiment is configured in the same manner as the first bend allowing section 8 of the inner tube body 6 according to the first embodiment. That is, the first bend allowing section 73 is formed of a first notch portion 74 formed in a side surface of the inner tubular member 70. The first notch portion 74 has two slit strings (a first slit string 75A and a second slit string 76A) formed in an outer peripheral surface of the distal end portion of the inner tubular member 70. The first slit string 75A and the second slit string 76A are substantially equal to the first slit string 10A and the second slit string 11A.

The first slit string 75A is constituted by aligning a plurality of substantially C-shaped slits 75 in the axial direction of the inner tubular member 70. In more detail, slits 75 are formed in along the axial direction of the inner tubular member 70 in one side of the radial direction of the inner tubular member 70 and in a side surface of the inner tubular member 70. Further, each slit 75 is arranged (extended) in the direction orthogonal to the axial direction of the inner tubular member 70. In slits 75, an appropriate groove width is t1 (not shown), and each length (pitch) p1 (not shown) between the adjacent slits 75 is aligned at a predetermined pitch. These slits 75 are opened in the same direction, respectively. These slits 75 form a slit group. Furthermore, each slit 75 is formed to be slightly larger than a substantially semicircular part (a part of 180°) of a circular cross section of the inner tubular member 70.

The second slit string 76A is constituted by aligning a plurality of substantially C-shaped slits 76 in the axial direction of the inner tubular member 70 like the first slit string 75A. In more detail, slits 76 are formed in along the axial direction of the inner tubular member 70 in the other side in the radial direction of the inner tubular member 70 and in a side surface of the inner tubular member 70. Furthermore, each slit 76 is arranged (extended) in the direction orthogonal to the axial direction of the inner tubular member 70. In slits 76, an appropriate groove width is t2 (not shown), and each length (pitch) p2 (not shown) between the adjacent slits 76 is aligned at a predetermined pitch. These slits 76 are opened in the same direction, respectively. These slits 76 form a slit group. Furthermore, each slit 76 is formed to be slightly larger than a substantially semicircular part (a part of 180°) of a circular cross section of the inner tubular member 70 like each slit 75 in the first slit string 75A (see FIG. 15).

It is to be noted that the number of slits 75 is equal to the number of slits 76 and t1=t2 and p1=p2 are achieved. Moreover, slits 75 and slits 76 are aligned (staggered) at an intervals of a half pitch. That is, slits 75 and slits 76 are aligned in the axial direction of the inner tubular member 70 in the staggered pattern. Additionally, slits 79 and slits 80 are not arranged on the same straight line in the radial direction of the inner tubular member 70.

Further, a second bend allowing section 77 that allows bending in a predetermined direction is provided on a side surface of a distal end portion of the outer tubular member 71. In this embodiment, the second bend allowing section 77 is formed of a second notch portion 78 formed in the side surface of the outer tubular member 71. The second notch portion 78 has two slit strings (a first slit string 79A and a second slit string 80A) formed in an outer peripheral surface of the distal end portion of the outer tubular member 71.

The first slit string 79A is constituted by aligning a plurality of substantially C-shaped slits 79 along the axial direction of the outer tubular member 71. In more detail, slits 79 are formed in along the axial direction of the outer tubular member 71 in one side in the radial direction of the outer tubular member 71 and in a side surface of the outer tubular member 71. Furthermore, each slit 79 is arranged (extended) in the direction orthogonal to the axial direction of the outer tubular member 71. In slits 79, an appropriate groove width is t3, and each length (pitch) p3 between the adjacent slits 79 is aligned at a predetermined pitch. These slits 79 are opened in the same direction, respectively. These slits 79 form a slit group. Moreover, each slit 79 is formed to be slightly larger than a substantially semicircular part (a part of 180°) of a circular cross section of the inner tubular member 70.

The second slit string 80A is constituted by aligning a plurality of substantially C-shaped slits 80 along the axial direction of the outer tubular member 71 like the first slit string 79A. In more detail, slits 80 are formed in along the axial direction of the outer tubular member 71 in the other side in the radial direction of the outer tubular member 71 and in the side surface of the outer tubular member 71. Furthermore, each slit 80 is arranged (extended) in the direction orthogonal to the axial direction of the outer tubular member 71. In slits 80, an appropriate groove width is t4, and each length (pitch) p4 between the adjacent slits 80 is aligned at a predetermined pitch. These slits 80 are opened in the same direction, respectively. These slits 80 form a slit group. Moreover, each slit 80 is formed to be slightly larger than a substantially semicircular part (a part of 180°) of a circular cross section of the inner tubular member 71 like each slit 76 in the second slit string 76A.

It is to be noted that the number of slits 79 is equal to the number of slits 80 and t3=t4 and p3=p4 are achieved. Additionally, slits 79 and slits 80 are aligned (staggered) at an intervals of a half pitch. That is, slits 79 and slits 80 are aligned in the axial direction of the outer tubular member 71 in the staggered pattern. Additionally, slits 79 and slits 80 are not arranged on the same straight line in the radial direction of the outer tubular member 71.

Further, in this embodiment, slits 75 and slits 76 are arranged at positions associated with slits 79 and slits 80 and formed with the same sizes with respect to slits 79 and slits 80.

The proximal end portion of the outer tubular member 71 is fixed to the proximal end portion of a housing 66a of the operating section 66. Moreover, the proximal end portion of the inner tubular member 70 is coupled with a non-illustrated coupling member disposed to the inside of the operation ring 69 of the housing 66a. As a result, when the operation ring 69 rotates in the periaxial direction of the insertion section 62, the inner tubular member 70 is operated to rotate in the periaxial direction with respect to the outer tubular member 71. When the operation ring 69 rotates, a rotational angle of the inner tubular member 70 in the periaxial direction of the insertion section 62 varies in the range of, e.g., a fixed position of 0° to a rotational position of 90°.

Moreover, when the operation ring 69 is held at the fixed position where the rotational angle is 0°, the distal end portion (the bending section 64) of the insertion section 62 is held at a first position where the first notch portion 74 (slits 75 and 76) of the inner tubular member 70 overlaps the second notch portion 78 (slits 79 and 80) of the outer tubular member 71. At this time, the distal end portion of the insertion section 62 changes to a state that it is apt to bend in a predetermined direction deviated from the axial direction of the insertion section 62 (the soft state) from a state that it is linearly stretched along the axial direction of the insertion section 62. The predetermined direction means one of the direction of the second slit string 80A of the outer tubular member 71 and the direction of the first slit string 79A of the same.

Additionally, when the rotational angle is 90°, the distal end portion of the insertion section 62 is held (switched) at a second position where the first notch portion 74 (slits 75 and 76) of the inner tubular member 70 does not overlap the second notch portion 78 (slits 79 and 80) of the outer tubular member 71. At this time, the distal end portion of the insertion section 62 is held in such a manner that a part of a tube wall of the inner tubular member 70 overlaps the inside of each slit 80 of the outer tubular member 71 and that a part of the tube wall of the inner tubular member 70 overlaps the inside of each slit 79 of the outer tubular member 71. In this case, the distal end portion of the insertion section 62 is in a state that it is hardly bent in a predetermined direction deviated from the axial direction of the insertion section 62 (a hard state).

In this manner, the operation ring 69 operates the inner tubular member 70 to rotate in the periaxial direction with respect to the outer tubular member 71 and thereby adjusts a relative positional relationship between the first bend allowing section 73 and the second bend allowing section 77, whereby the bendability of the bending section 64 constituted of the outer tubular member 71 and the inner tubular member 70 is changed.

Further, one small circular hole 71b1 is arranged at a central position of slit 79 of the outer tubular member 71. Likewise, the other small circular hole 71b2 is arranged at a central position of slit 80 of the outer tubular member 71. Operation wires 81a and 81b that operates the bending section 64 to bend are inserted into these small circular holes 71b1 and 71b2, respectively.

A distal end portion of the operation wire 81a is fixed to the outer tubular member 71 in the vicinity of an end portion of the small circular hole 71b1. Likewise, a distal end portion of the operation wire 81b is fixed to the outer tubular member 71 in the vicinity of an end portion of the small circular hole 71b2. Proximal end portions of these operation wires 81a and 81b are coupled with a non-illustrated bending operation mechanism incorporated in the operating section 66. A bending lever 68 is fixed to the bending operation mechanism. One of the operation wires 81a and 81b is operated to be pulled through the bending operation mechanism in accordance with an operation of this bending lever 68. Furthermore, the bending section 64 is operated to bend in response to an operation of the pulled operation wire 81a or operation wire 81b.

A function of the above-described configuration will now be described. When the endoscope 61 according to this embodiment is used, the operation ring 69 rotates in the periaxial direction of the insertion section 62. As a result, the bending section 64 is changed to one of an unbendable state (a hard state) and a bendable state (a soft state).

When the endoscope 61 according to this embodiment is used, the bending section 64 is held at the fixed position where the rotational angle of the operation ring 69a is 0°. In this case, the distal end portion (the bending portion 64) of the insertion section 62 is held at the first position where the first notch portion 74 of the inner tubular member 70 overlaps the second notch portion 78 of the outer tubular member 71. In this state, the bending section 64 is changed from the state that the bending section 64 is linearly stretched along the axial direction of the insertion section 62 as shown in FIG. 13 and held in the state that the bending section 64 can readily bend in a predetermined direction deviated from the axial direction of the insertion section 62 (the soft state). Therefore, in this state, when one of the operation wires 81a and 81b is operated to be pulled by an operation of the bending lever 68, the bending section 64 is operated to bend.

In this state, the insertion section 62 is guided into the renal pelvis from the urinary bladder through the ureter. At this time, when the insertion section 62 is pushed and inserted into the urethral orifice, the bending section 64 is apt to bend due to insertion resistance during the inserting operation. In this state, the operation of inserting the insertion section 62 of the endoscope 61 such as a ureteral scope straight into the orifice of a patient along the axial direction is hard to be performed.

Therefore, in this embodiment, before the insertion section 62 is inserted into the urethral orifice, the operation ring 69 is rotated to the rotational position of 90° in the periaxial direction of the insertion section 62. As a result, the bending section 4 varies to the unbendable state (the hard state).

When the rotational angle is 90°, the insertion section 62 (the bending section 64) is held (switched) at the second position where the first notch portion 74 of the inner tubular member 70 does not overlap the second notch portion 78 of the outer tubular member 71. At this time, the distal end portion of the insertion section 62 is held in a state that a part of the tube wall of the inner tubular member 70 overlaps the inside of each slit 80 of the outer tubular member 71 and a state that a part of the tube wall of the inner tubular member 70 overlaps the inside of each slit 79 of the outer tubular member 71. In this state, the distal end portion of the insertion section 62 enters the state that the distal end portion of the insertion section 62 is hard to bend in a predetermined direction deviated from the axial direction of the insertion section 62 (the hard state).

Then, the insertion section 62 is operated to be inserted into the ureteral orifice. At this time, when the insertion section 62 is pushed and inserted into the ureteral orifice, the bending section 64 is hard to bend even though it undergoes the insertion resistance during the inserting operation. Therefore, in this state, the operation of inserting the insertion section 62 straight into the orifice of the patient is easily carried out. As a result, at the time of an operation of guiding the insertion section 62 into the renal pelvis from the urinary bladder through the ureter, it is not necessary to additionally use a guide sheath and the like or use a device that is inserted into the tube to fix the shape like a stilet.

Therefore, in this embodiment, as compared with an example where the guide sheath or the like is additionally utilized to improve push-in performance of the insertion section 62 into the orifice of the patient and the insertion section 62 is thereby inserted, the number of devices required for insertion of the insertion section 62 is not increased, and the operation of inserting the insertion section 62 into the body cavity is simplified.

Thus, the above-described configuration exercises the following effects. That is, in the endoscope 61 such as a ureteral scope according to this embodiment, when inserting the insertion section 62 into the body cavity, the bendability of the distal end portion (the bending section 64) of the insertion section 62 can be changed as required. Therefore, as compared with the example where the guide sheath or the like is additionally utilized to improve the push-in performance of the insertion section 62 and the insertion section 62 is thereby inserted, the operation of inserting the insertion section 62 into the body cavity can be simplified without increasing the number of devices required for the insertion of the insertion section 62 in this embodiment.

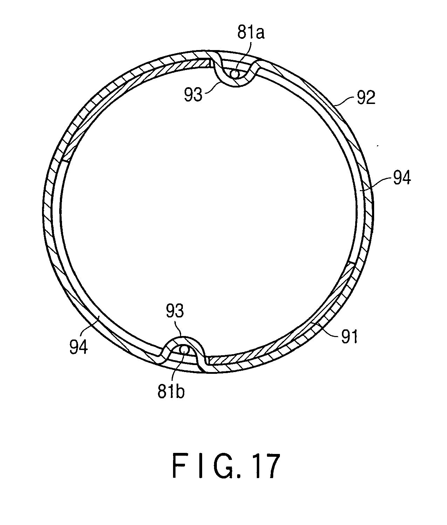

Further, FIG. 16 and FIG. 17 shows a fifth embodiment according to the present invention. This embodiment is obtained by changing the configuration of the bending section 64 according to the fourth embodiment (see FIG. 13, FIG. 14, and FIG. 15) as follows.

That is, a bending section 64 according to this embodiment is configured in substantially the same manner as the bending change section 4 of the medical flexible section 1 according to the first embodiment. Here, an inner tubular member 91 of an insertion section 62 is formed of a metallic circular tube member. An outer tubular member 92 is likewise formed of a metallic circular tube member. An inside diameter of the outer tubular member 92 is slightly larger than an outside diameter of the inner tubular member 91. As a result, a clearance is formed between the outer tubular member 92 and the inner tubular member 91. The clearance has a size that enables the inner tubular member 91 to rotate in the periaxial direction with respect to the outer tubular member 92.

As shown in FIG. 17, in this embodiment, two wire guides 93 are formed at part of the outer tubular member 92. Each wire guide 93 is provided to protrude toward the inner side of the outer tubular member 92. The two wire guides 93 are arranged at positions that are 180° apart in the circumferential direction of the outer tubular member 92.

Two clearance grooves 94 are formed in the inner tubular member 91 at positions associated with the two wire guides 93 of the outer tubular member 92. Each clearance groove 94 is extended to a rotational position of substantially 90° in the circumferential direction.

Each wire guide 93 is protruded to the inner side of the inner tubular member 91 through the clearance groove 94. Moreover, an operation wire 81a or 81b that operates a bending section 64 to bend is inserted into the wire guide 93.

A function of the above-described configuration will now be described. When the endoscope 61 according to this embodiment is used, the rotational angle of an operation ring 69 is held at a fixed position of 0°. In this case, a distal end portion (the bending portion 64) of the insertion section 62 is held at a first position where a first notch portion 95 of the inner tubular member 91 overlaps a second notch portion 96 of the outer tubular member 92 (see FIG. 16). In this state, the bending section 64 is changed from the state that the bending section 64 is linearly stretched along the axial direction of the insertion section 62 as shown in FIG. 16 and held in the state that the bending section 64 can readily bend in a predetermined direction deviated from the axial direction of the insertion section 62 (the soft state). The predetermined direction means one of a direction of a second slit string 98A of the outer tubular member 92 and a direction of a first slit string 97A of the same. Therefore, in this state, when one of the operation wires 81a and 81b is operated to be pulled by an operation of a bending lever 68, the bending section 64 is operated to bend.

When the rotational angle is 90°, the insertion section 62 (the bending section 64) is held (switched) at a second position where the first notch portion 95 of the inner tubular member 91 does not overlap the second notch portion 96 of the outer tubular member 92. At this time, the distal end portion of the insertion section 62 is held in a state that a part of a tube wall of the inner tubular member 91 overlaps the inside of each slit 98 of the outer tubular member 92 and a state that a part of the tube wall of the inner tubular member 91 overlaps the inside of each slit 97 of the outer tubular member 92. In this state, the distal end portion of the insertion section 62 enters the state that the distal end portion of the insertion section 62 is hard to bend in a predetermined direction deviated from the axial direction of the insertion section 62 (the hard state).

Thus, the above-described configuration exercises the following effects. That is, in the endoscope 61 such as a ureteral scope according to this embodiment, when inserting the insertion section 62 into the body cavity, the bendability of the distal end portion (the bending section 64) of the insertion section 62 can be changed as required. Therefore, as compared with the example where a guide sheath or the like is additionally utilized to improve the push-in performance of the insertion section 62 and the insertion section 62 is thereby inserted, the operation of inserting the insertion section 62 into the body cavity can be simplified without increasing the number of devices required for the insertion of the insertion section 62 in this embodiment.

Moreover, the present invention is not restricted to the foregoing embodiments. For example, the medical flexible section 1 may be a guide sheath or a medical instrument such as a cannula used for ERCP (endoscopic retrograde cholangiopancreatography).

Additionally, the first notch portion 9 of the inner tube body 6 in the medical flexible section 1 according to the first embodiment (see FIGS. 1 to 9) may be larger than the second notch portion 13 of the outer tube body 7, the second notch portion 13 may be exposed from the first notch portion 9 at the first position, and at least a part of the side surface of the outer tube body 7 in which the second notch portion 13 is not formed may be exposed from the first notch portion 9 at the second position.

Further, the first notch portion 9 may be smaller than the second notch portion 13, the first notch portion 9 may be placed above the second notch portion 13 at the first position, and at least a part of the first notch portion 13 may be placed on the side surface of the outer tube body 7 in which the second notch portion 13 is not formed at the second position. Furthermore, the intervals for the formation of the first notch portion 9 (the pitch between the notches) on the inner tube body 6 do not have to coincide with the intervals for the formation of the second notch portions 13 on the outer tube body 7, and the bending may be allowed by adjusting a diameter difference (setting a large diameter difference) between the inner tube body 6 and the outer tube body 7 when the position of the first notch portion 9 deviates from the position of the second notch portion 13 in the axial direction.

Moreover, the inner tubular member 70 according to the fourth embodiment (FIG. 13, FIG. 14, and FIG. 15) may have a first bend allowing section 73 constituted of a first notch portion 74 formed in the side surface of the inner tubular member 70 and a first bending regulating section formed of a sidewall surface in which the first notch portion 74 is not formed, and the outer tubular member 71 may have a second bend allowing section 77 constituted of a second notch portion (78) formed in the side surface of the outer tubular member 71 and a second bending regulating section formed of a sidewall surface in which the second notch portion is not formed.

Additionally, the present invention can be of course modified in many ways without departing from the scope of the invention.

Other characteristic technical matters of this application will be additionally written as follows.

### Note

### (Additional note 1)

A medical flexible section (1) comprising:
an inner tube body (6, 22, 41) which has a first bend allowing section (8, 27, 43) configured to allow bending in a predetermined direction provided on a side surface thereof and which forms a duct;
an outer tube body (7, 21, 42) which has a second bend allowing section (12, 23, 47) configured to allow bending in a predetermined direction provided on a side surface thereof and in which the inner tube body (6, 22, 41) is inserted; and
an operating section (5) configured to operate the inner tube body (6, 22, 41) in such a manner that the inner tube body (6, 22, 41) is moved with respect to the outer tube body (7, 21, 42) to one of a first position where the first bend allowing section (8, 27, 43) overlaps the second bend allowing section (12, 23, 47) and a second position where the first bend allowing section (8, 27, 43) does not overlap the second bend allowing section (12, 23, 47).

### (Additional note 2)

In Additional note 1,
the medical flexible section (1),
wherein the first bend allowing section (8, 27, 43) has a first notch portion (9, 28, 45) formed in the side surface of the inner tube body (6, 22, 41), and
the second bend allowing section (12, 23, 47) has a second notch portion (13, 24, 49) formed in the side surface of the outer tube body (7, 21, 42).

### (Additional note 3)

In Additional note 2,
the medical flexible section (1),
wherein the first notch portion (the inner side) (9, 28, 25) is larger than the second notch portion (the outer side) (13, 24, 49),
the second notch portion (13, 24, 49) is exposed from the first notch portion (9, 28, 45) at the first position, and
at least a part of the side surface of the outer tube body (7, 21, 42) in which the second notch portion (13, 24, 49) is not formed is exposed from the first notch portion (9, 28, 45) at the second position.

### (Additional note 4)

In Additional note 2,
the medical flexible section (1),
wherein the first notch portion (the inner side) (9, 28, 45) is smaller than the second notch portion (the outer side) (13, 24, 49),
the first notch portion (9, 28, 45) is placed above the second notch portion (13, 24, 49) at the first position, and
at least a part of the first notch portion (9, 28, 45) is placed on the side surface of the outer tube body (7, 21, 42) in which the second notch portion (13, 24, 49) is not formed at the second position.

### (Additional note 5)

In Additional note 2,
the medical flexible section (1),
wherein the first notch portion (9, 28, 45) and the second notch portion (13, 24, 49) have substantially the same size, and
a position of the first notch portion (9, 28, 45) substantially coincides with a position of the second notch portion (13, 24, 49) at the first position, and a position of the first notch portion (9, 28, 45) is different from a position of the second notch portion (13, 24, 49) at the second position.

### (Additional note 6)

In Additional note 1, the medical flexible section (1), wherein the inner tube body (6, 22, 41) is inserted in the outer tube body (7, 21, 42) to be rotatable on a long axis direction in such a manner that the inner tube body (6, 22, 41) can be placed at least at the first position and the second position.

### (Additional note 7)

In Additional note 6,
the medical flexible section (1),
wherein the first notch portion (9, 28) is constituted of a plurality of slits (10, 11, 25, 26) which are aligned at a predetermined pitch and opened in the same direction, respectively, and
the second notch portion (13, 24) is constituted of a plurality of slits (14, 15, 29, 30) which are aligned at a pitch substantially equivalent to that of the first notch portion (9, 28) and opened in the same direction, respectively.

### (Additional note 8)

An insertion section (62) of a medical apparatus (61), comprising:
a first tubular member (70) which has a first bend allowing section (73) configured to allow bending and a first bending regulating section configured to regulate bending at positions except the first bend allowing section (73) formed on a side surface thereof;
a second tubular member (71) which has a second bend allowing section (77) configured to allow bending and a second bending regulating section configured to regulate bending at positions except the second bend allowing section (77) formed on a side surface thereof and in which the first tubular member is inserted; and
a switching section (69) configured to switch the first tubular member (70) with respect to the second tubular member (71) to one of a first state that bending of the first tubular member (70) and the second tubular member (71) is allowed when a position of the first bend allowing section (73) coincides with a position of the second bend allowing section (77) and a second state that the bending of the first tubular member (70) and the second tubular member (71) is regulated when a position of the first bend allowing section (73) coincides with a position of the second bending regulating section or a position of the second bend allowing section (77) coincides with a position of the first bending regulating section.

### (Additional note 9)

In Additional note 8,
the insertion section (62) of the medical apparatus (61),
wherein the first bend allowing section (73) has a first notch portion (74) formed in a side surface of the first tubular member (70),
the second bend allowing section (77) has a second notch portion (78) formed in a side surface of the second tubular member (71),
the first bending regulating section is constituted of the side surface of the first tubular member (70) in which the first notch portion (74) is not formed, and
the second bending regulating section is constituted of the side surface of the second tubular member (71) in which the second notch portion (78) is not formed.

### (Additional note 10)

An insertion section of a medical apparatus, comprising:
an inner tube (a pipe, a sheath, a flexible section) in which a plurality of slits are provided in a predetermined direction (position) at predetermined intervals; and
an outer tube in which slits are provided at the same intervals as those of the slits in the inner tube,
wherein the inner tube is inserted in the outer tube to allow its swiveling movement on a long axis direction along a circumferential direction, and positions of the slits in the inner and outer tubes coincide with each other at a predetermined rotational position.

The present invention is effective for the technical field of the medical flexible section which is used by inserting an insertion section of a medical apparatus having a small diameter like a medical instrument such as a ureteral scope or a cannula in endoscopic retrograde cholangiopancreatography (ERCP) into a body cavity and the insertion section of the medical apparatus.

Although the preferred embodiments and modifications of the present invention have been described, the present invention is not restricted thereto. These embodiments and modifications can be combined in many ways.

## Claims

1. A medical flexible section comprising:
an inner tube body which has a first bend allowing section configured to allow bending in a predetermined direction provided on a side surface thereof and forms a duct;
an outer tube body which has a second bend allowing section configured to allow bending in a predetermined direction provided on a side surface thereof and in which the inner tube body is inserted; and
a change section configured to change bendability of an insertion section constituted of the outer tube body and the inner tube body by rotating the inner tube body in a periaxial direction with respect to the outer tube body to adjust a relative positional relationship between the first bend allowing section and the second bend allowing section.

2. The medical flexible section according to claim 1,
wherein the first bend allowing section has a first notch portion formed in the side surface of the inner tube body, and
the second bend allowing section has a second notch section formed in the side surface of the outer tube body.

3. The medical flexible section according to claim 2,
wherein the change section operates the inner tube body to move between a first position where the first notch portion and the second notch portion are placed in substantially the same rotating direction and a second position where the first notch portion and the second notch portion are placed in different rotational directions when the change section operates the inner tube body to rotate in a periaxial direction with respect to the outer tube body.

4. The medical flexible section according to claim 2,
wherein the first notch portion has a slit group, and the slit group has a plurality of slits which are aligned in the side surface of the inner tube body along an axial direction of the inner tube body at a predetermined pitch, opened in the same direction, and orthogonal to the axial direction of the inner tube body, and
the second notch portion has a slit group, and the slit group has a plurality of slits which are aligned in the side surface of the outer tube body along an axial direction of the outer tube body at a pitch equivalent to that of the first notch portion, opened in the same direction, and orthogonal to the axial direction of the outer tube body.

5. The medical flexible section according to claim 3,
wherein the second position is a rotational position to which the inner tube body is rotated 90° in the periaxial direction from the first position.

6. The medical flexible section according to claim 1,
wherein the first bend allowing section has:
a first notch portion arranged in the side surface of the inner tube body along the axial direction of the inner tube body; and
a first buried member which is formed of a material different from the inner tube body in hardness and placed in the first notch portion,
the second bend allowing section has:
a second notch portion extended in the side surface of the outer tube body along the axial direction of the outer tube body; and
a second buried member which is formed of a material different from the outer tube body in hardness and placed in the second notch portion, and
the change section moves the inner tube body between a first position at which the first buried member of the inner tube body is placed in substantially the same rotational direction as that of the second buried member and a second position at which the first buried member is placed in a rotational direction different from that of the second buried member when the change section operates the inner tube body to rotate in the periaxial direction with respect to the outer tube body.

7. The medical flexible section according to claim 6,
wherein the inner tube body is formed of a hard resin material,
the first buried member is formed of a resin material which is of the same type as the inner tube body and softer than the inner tube body,
the outer tube body is formed of a hard resin material, and
the second buried member is formed of a resin material which is of the same type as the outer tube body and softer than the outer tube body.

8. An insertion section of a medical apparatus, comprising: a bending section arranged at a distal end portion of the insertion section inserted into a body cavity; an operating section arranged at a proximal end portion of the insertion section; and a bending operating section configured to bend the bending section at the operating section,
wherein the insertion section comprises: an outer tubular member; and an inner tubular member which is inserted in the outer tubular member to be rotatable in a periaxial direction,
the inner tubular member has a first bend allowing section configured to allow bending in a predetermined direction at a distal end portion thereof, and the outer tubular member has a second bend allowing section configured to allow bending in a predetermined direction at a distal end portion thereof,
the bending section is formed by assembling the first bend allowing section and the second bend allowing section, and
the insertion section comprises a change section which is arranged in the operating section and changes bendability of the bending section by rotating the inner tubular member in the periaxial direction with respect to the outer tubular member to adjust a relative positional relationship of the first bend allowing section and the second bend allowing section.

9. The insertion section of a medical apparatus according to claim 8,
wherein the inner tubular member comprises: a first bend allowing section constituted of a first notch portion formed in the side surface of the inner tubular member; and a first bending regulating section constituted of a sidewall surface in which the first notch portion is not formed, and
the outer tubular member comprises: a second bend allowing section constituted of a second notch portion formed in the side surface of the outer tubular member; and a second bending regulating section constituted of a sidewall surface in which the second notch portion is not formed.

10. The insertion section of a medical apparatus according to claim 8,
wherein a wire guide is provided on the outer tubular member to protrude toward the inner side,
a clearance groove, which is configured to avoid interference with the wire guide when the inner tubular member rotates in the periaxial direction, is formed in the inner tubular member,
a distal end portion of an operation wire configured to bend the bending section is fixed to a distal end portion of the bending section, and a proximal end of the operation wire is extended to the operating section side through the wire guide and coupled with the bending operating section, and
the operation wire is pulled and driven to bend the bending section when the bending operating section is operated.
